# EUROPEAN PATENT APPLICATION

(11) **EP 4 282 860 A1**
(43) Date of publication of application: **29.11.2023**
(21) Application number: 22742216.9
(22) Date of filing: 20.01.2022
(51) Int. Cl.: C07D 263/57, C07D 413/14, A61K 31/4995, A61K 31/454, A61P 31/16

(54) **CRYSTAL FORM OF ANTI-INFLUENZA VIRUS COMPOUND, PREPARATION METHOD FOR CRYSTAL FORM, AND USE OF CRYSTAL FORM**

(30) Priority: 22.01.2021 CN 202110088776
(71) Applicant: Sichuan Haisco Pharmaceutical Co., Ltd., Sichuan 611130 (CN)
(72) Inventor: LI, Yao, Chengdu, Sichuan 611130 (CN); ZHANG, Guobiao, Chengdu, Sichuan 611130 (CN); ZHANG, Xiaobo, Chengdu, Sichuan 611130 (CN)
(74) Representative: Casalonga
(86) International application number: PCT/CN2022/072933
(87) International publication number: WO 2022/156737

(57) **Abstract**

Disclosed are a crystal form of a compound (1*S*,2*S*)-2-fluoro-N-(2-(2-(4-((*R*)-(5-methyl-2H-tetrazol-2-yl)(phenyl)methyl)piperidine-1-formyl)pyridin-4-yl)benzo[d]oxazol-5-yl)cyclopropyl-1-carboxamide having a structure of formula (A), a preparation method for the crystal form, and a use of the crystal form in the preparation of a drug for treating and/or preventing influenza.

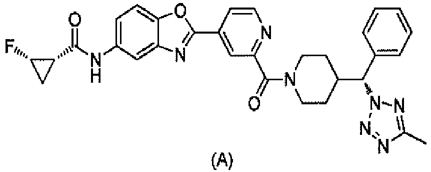

## Description

The present application is based on and claims the right of priority for the application with the application no. being CN 2021 10088776.3 and the filing date being 22 January 2021, and the disclosure of the present application is incorporated herein by reference in its entirety.

### Technical Field

The present application relates to a crystal form of a compound (1*S*,2*S*)-2-fluoro-N-(2-(2-(4-((*R*)-(5-methyl-2H-tetrazol-2-yl)(phenyl)methyl)piperidine-1-carbonyl)pyridin-4-yl)benzo[d]oxazol-5-yl)cyclopropane-1-carboxamide, and a preparation method therefor and the medical use thereof.

### Background Art

Influenza (flu for short) is an acute respiratory infection caused by influenza viruses and is also a highly infectious and fast-transmitting disease. At present, anti-influenza virus drugs mainly target the hemagglutinin receptor, neuraminidase and matrix protein of the viral envelope. PCT/CN 2020/110764 has reported a series of pyridine derivatives, which play a role in preventing and treating influenza by means of binding to the conserved region of hemagglutinin (HA), wherein (1*S*,2*S*)-2-fluoro-N-(2-(2-(4-((*R*)-(5-methyl-2H-tetrazol-2-yl)(phenyl)methyl)piperidine-1-carbonyl)pyridin-4-yl)benzo[d]oxazol-5-yl)cyclopropane-1-carboxamide, i.e., a compound of formula (A), exhibits a good anti-influenza virus activity and has potential to prevent and/or treat influenza.

The structure of a crystal form as a pharmaceutically active ingredient often affects the chemical stability, the degree of difficulty in preparation, purity and even bioavailability of a drug. Different crystallization conditions and storage conditions may lead to the change in the crystal form structure of a compound, and sometimes may be accompanied by the formation of other forms of crystal forms. Generally, amorphous pharmaceutical products have no regular crystal form structure, and often have other defects, such as poor stability of the product, fine crystallization, difficulty in filtration, easy caking, poor fluidity, etc. Polymorphic forms of drugs have different requirements for product storage, production and scale-up production. Therefore, it is necessary to study the crystal form of the compound of formula (A) (PCT/CN 2020/110764 recited an amorphous form of the compound (A)) and the related preparation methods in-depth to improve various properties of the compound of formula (A).

### Summary of the Invention

The present application provides a crystal form of a compound of formula (A), a method for preparing the crystal form, a composition and the use thereof in the preparation of a drug for preventing/treating influenza. The crystal forms provided by the present application are high in purity and bioavailability and good in pressure stability, which are beneficial to the preparation of medicaments; and especially a crystal form I has more excellent stability, higher bioavailability and significantly lower hygroscopicity compared with an amorphous form.

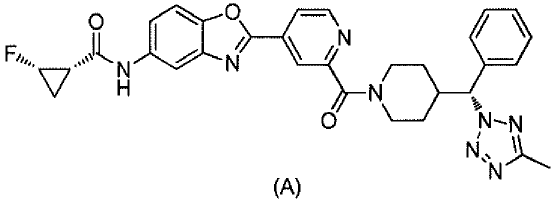

The present application first provides a crystal form I of a compound of formula (A), wherein the crystal form I has an X-ray powder diffraction pattern comprising characteristic diffraction peaks at 4.1° ± 0.2°, 10.1° ± 0.2°, 14.8° ± 0.2°, 17.5° ± 0.2°, 20.4° ± 0.2°, 21.2° ± 0.2°, and 23.7° ± 0.2° 20, as determined by using Cu-Kα radiation.

Furthermore, the crystal form I of the compound of formula (A) has an X-ray powder diffraction pattern further comprising characteristic diffraction peaks at 19.5° ± 0.2°, 22.7° ± 0.2°, 24.4° ± 0.2°, 25.6° ± 0.2°, 26.9° ± 0.2°, and 28.7° ± 0.2° 2θ.

Furthermore, the crystal form I of the compound of formula (A) has an X-ray powder diffraction pattern further comprising characteristic diffraction peaks at14.2° ± 0.2°, 16.3° ± 0.2°, 18.3° ± 0.2°, 18.6° ± 0.2°, and 24.0° ± 0.2° 2θ.

Furthermore, the crystal form I of the compound of formula (A) has an X-ray powder diffraction pattern comprising characteristic diffraction peaks at 2θ in the table below:

| Number | 20 |
|---|---|
| 1 | 4.1° ± 0.2° |
| 2 | 10.1° ± 0.2° |
| 3 | 11.3° ± 0.2° |
| 4 | 14.2° ± 0.2° |
| 5 | 14.8° ± 0.2° |
| 6 | 16.3° ± 0.2° |
| 7 | 16.5° ±0.2° |
| 8 | 16.8° ± 0.2° |
| 9 | 17.5° ± 0.2° |
| 10 | 18.3° ± 0.2° |
| 11 | 18.6° ± 0.2° |
| 12 | 19.5° ± 0.2° |
| 13 | 20.4° ± 0.2° |
| 14 | 21.2° ± 0.2° |
| 15 | 22.7° ±0.2° |
| 16 | 23.7° ±0.2° |
| 17 | 24.0° ±0.2° |
| 18 | 24.4° ±0.2° |
| 19 | 25.6° ±0.2° |
| 20 | 26.9° ±0.2° |
| 21 | 27.9° ±0.2° |
| 22 | 28.7° ± 0.2° |
| 23 | 29.9° ± 0.2° . |

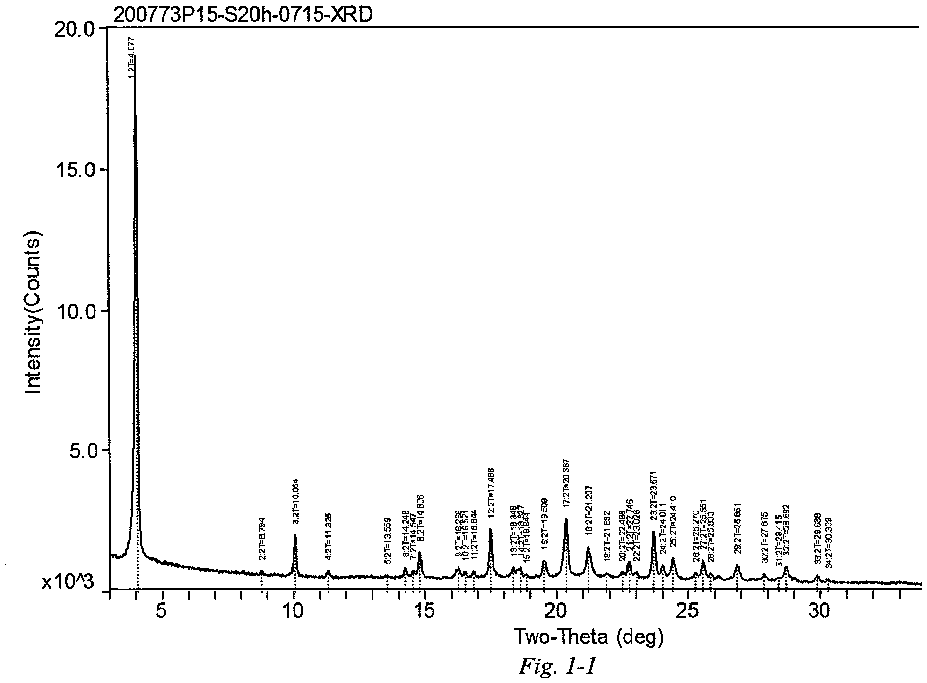

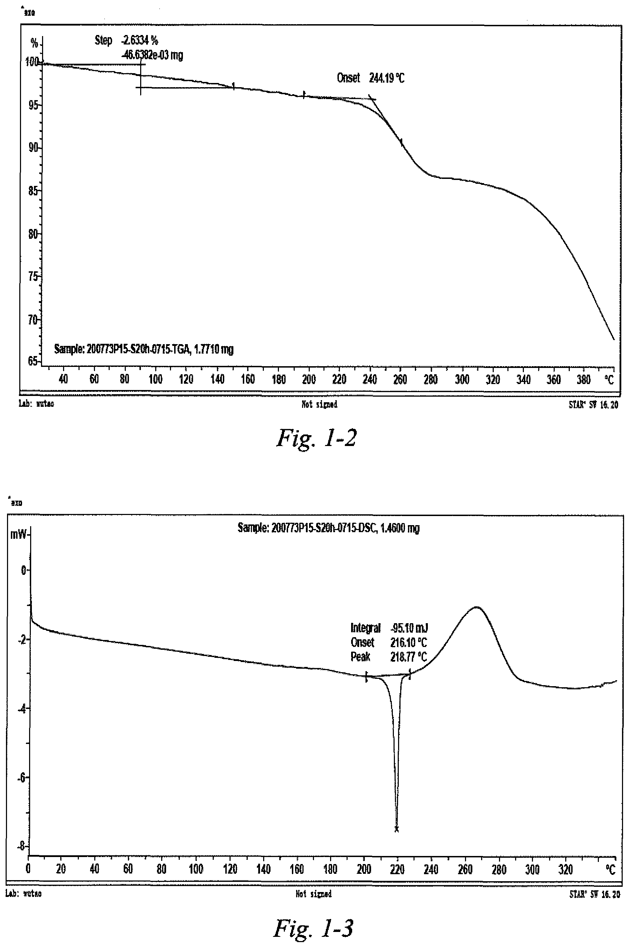

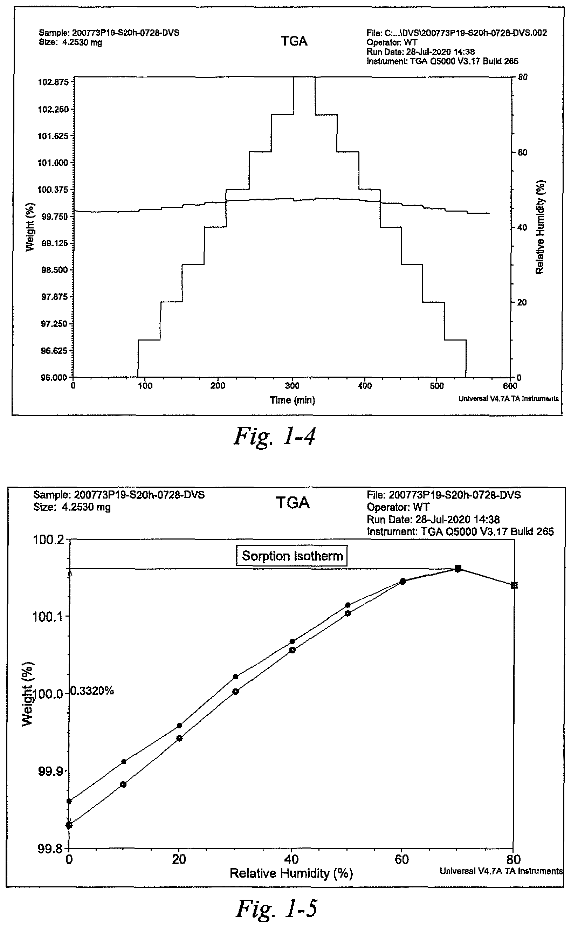

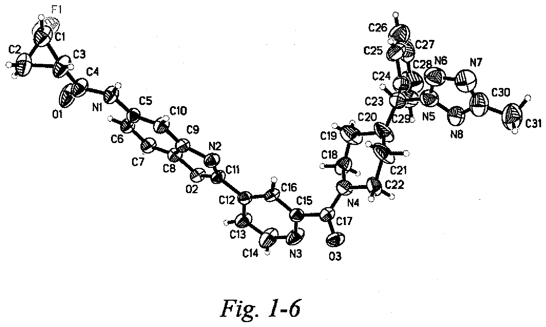

Furthermore, the crystal form I of the compound of formula (A) has an X-ray powder diffraction pattern substantially as shown in Figure 1-1.

Furthermore, the crystal form I of the compound of formula (A) has a melting point of 216°C ± 3°C and a decomposition temperature of 246°C ± 3°C.

Furthermore, the crystal form I of the compound of formula (A) has a TGA that represents a slow loss of 2.6% ± 0.3% of weight before 150°C, indicating an anhydride or a tunnel hydrate.

Furthermore, the crystal form I of the compound of formula (A) has a TGA curve substantially as shown in Figure 1-2.

Furthermore, the crystal form I of the compound of formula (A) has a DSC pattern substantially as shown in Figure 1-3.

Furthermore, the crystal form I of the compound of formula (A) has a TGA curve substantially as shown in Figure 1-2 and a DSC pattern substantially as shown in Figure 1-3.

The present application also provides a crystal form of a compound of formula (A), which has the following unit cell parameters when characterized using single crystal structure information:
crystal system: monoclinic system;
space group: C2;
cell parameters: a = 46.5183(13) Å, b = 6.6246(3) Å, c = 20.2880(5) Å, α = γ = 90°, β = 109.7650(10)°;
crystal axis ratios: a/b = 7.0221 b/c = 0.3265 c/a = 0.4361;
Z: 8;
cell volume: 5883.7(3) Å³;
theoretical density: 1.311 g/cm³.

The present application also provides a method for preparing the crystal form I of the compound of formula (A), comprising:
1) a magma crystallization process, which involves: at 4°C - 40°C, adding a crude of the compound of formula (A) to a solvent to obtain a suspension, and stirring and separating the suspension to obtain the crystal form 1; or
2) a diffusion crystallization process, which involves: taking and placing a crude of the compound of formula (A) in a centrifugal tube, adding a solvent 1 and dissolving into a clear solution, placing the clear solution in the solvent atmosphere of a solvent 2 and leaving same to stand for a period of time, precipitating out a solid, centrifuging and drying to obtain the crystal form I.

The present application also provides a method for preparing the crystal form I of the compound of formula (A), comprising:
1) a magma crystallization process, which involves: at 4°C - 40°C, adding a crude of the compound of formula (A) to a solvent to obtain a suspension, and stirring and separating the suspension to obtain the crystal form I; or
2) a diffusion crystallization process, which involves: taking and placing a crude of the compound of formula (A) in a centrifugal tube, adding a solvent 1 and dissolving into a clear solution, placing the clear solution in the solvent atmosphere of a solvent 2 and leaving same to stand, precipitating out a solid, centrifuging and drying to obtain the crystal form I.

The present application also provides a method for preparing the crystal form I of the compound of formula (A), comprising:
1) at 4°C - 40°C, mixing a crude of the compound of formula (A) with a solvent to obtain a suspension, and stirring and separating the suspension to obtain the crystal form I; or
2) mixing a crude of the compound of formula (A) with a solvent 1 and dissolving into a clear solution, placing the clear solution in the solvent atmosphere of a solvent 2 and leaving same to stand, precipitating out a solid, centrifuging and drying to obtain the crystal form I.

In some embodiments, according to the above-mentioned methods for preparing the crystal form I, the solvent in 1) is a single-solvent system or a double-solvent mixed system, the single-solvent system is selected from one of isopropyl ether and methyl tert-butyl ether, the double-solvent mixed system comprises a first solvent and a second solvent, the first solvent is selected from one of methanol, ethanol, acetonitrile, toluene and isopropyl acetate, the second solvent is selected from one of water, n-heptane, n-butyl acetate and methyl tert-butyl ether.

In some embodiments, according to the above-mentioned methods for preparing the crystal form I, a volume ratio of the first solvent to the second solvent in the double-solvent mixed system in 1) is 6 : 1 - 1 : 5.

In some embodiments, according to the above-mentioned methods for preparing the crystal form I, the double-solvent mixed system in 1) is a methanol-water mixed solution, an ethanol-n-heptane mixed solution, an acetonitrile-water mixed solution, a toluene-n-butyl acetate mixed solution, an isopropyl acetate-methyl tert-butyl ether mixed solution or a toluene-methyl tert-butyl ether mixed solution.

In some specific embodiments, according to the above-mentioned methods for preparing the crystal form 1, 1) comprises: adding a crude of the compound of formula (A) to a methanol/water system, stirring magma at room temperature for 13 days, centrifuging and drying to obtain the crystal form I; or adding a crude of the compound of formula (A) to methyl tert-butyl ether, stirring the magma in a water bath at 40°C for 3 days, centrifuging and drying to obtain the crystal form I.

In some embodiments, according to the above-mentioned methods for preparing the crystal form I, the solvent 1 in 2) is an ester solvent, preferably n-butyl acetate; and the solvent 2 is an ether solvent, preferably isopropyl ether.

The present application also provides a crystal form II of the compound of formula (A), wherein the crystal form II has an X-ray powder diffraction pattern comprising characteristic diffraction peaks at 3.9° ± 0.2°, 7.7° ± 0.2°, 8.1° ± 0.2°, 10.4° ± 0.2°, 14.3° ± 0.2°, 15.3° ± 0.2°, 17.7°±0.2°, 18.3° ± 0.2°, 18.6° ± 0.2°, 21.0° ± 0.2°, 21.4° ± 0.2°, 23.4° ± 0.2°, 24.7° ± 0.2°, 26.1° ± 0.2°, and 27.6° ± 0.2° 2θ, as determined by using Cu-Kα radiation.

Furthermore, the crystal form II of the compound of formula (A) has an X-ray powder diffraction pattern further comprising characteristic diffraction peaks at 11.5° ± 0.2°, 16.9° ± 0.2°, 17.1° ± 0.2°, 20.0° ± 0.2°, 21.7° ± 0.2°, 22.2° ± 0.2°, 22.4° ± 0.2°, 25.1° ± 0.2°, and 30.8° ± 0.2° 2θ.

Furthermore, the crystal form II of the compound of formula (A) has an X-ray powder diffraction pattern further comprising characteristic diffraction peaks at 9.1° ± 0.2°, 14.7° ± 0.2°, 19.1° ± 0.2°, 19.6° ± 0.2°, 24.4° ± 0.2°, 26.4° ± 0.2°, 26.7° ± 0.2°, 27.1° ± 0.2°, 30.1° ± 0.2°, and 30.3° ±0.2° 2Θ.

Furthermore, the crystal form II of the compound of formula (A) has an X-ray powder diffraction pattern comprising characteristic diffraction peaks at 2θ in the table below:

| Number | 20 |
|---|---|
| 1 | 3.9° ± 0.2° |
| 2 | 7.7° ± 0.2° |
| 3 | 8.1° ± 0.2° |
| 4 | 9.1° ± 0.2° |
| 5 | 10.4° ±0.2° |
| 6 | 11.5° ± 0.2° |
| 7 | 14.3° ± 0.2° |
| 8 | 14.7° ± 0.2° |
| 9 | 15.3° ± 0.2° |
| 10 | 16.1° ±0.2° |

| | |
|---|---|
| 11 | 16.9° ±0.2° |
| 12 | 17.1° ± 0.2° |
| 13 | 17.7° ± 0.2° |
| 14 | 18.3° ± 0.2° |
| 15 | 18.6° ±0.2° |
| 16 | 19.1° ±0.2° |
| 17 | 19.6° ±0.2° |
| 18 | 20.0° ± 0.2° |
| 19 | 20.6° ±0.2° |
| 20 | 21.0° ± 0.2° |
| 21 | 21.4° ± 0.2° |
| 22 | 21.7° ± 0.2° |
| 23 | 22.2° ± 0.2° |
| 24 | 22.4° ± 0.2° |
| 25 | 23.4° ± 0.2° |
| 26 | 24.4° ± 0.2° |
| 27 | 24.7° ± 0.2° |
| 28 | 25.1° ± 0.2° |
| 29 | 26.1° ± 0.2° |
| 30 | 26.4° ± 0.2° |
| 31 | 26.7° ± 0.2° |
| 32 | 27.1° ± 0.2° |
| 33 | 27.6° ± 0.2° |
| 34 | 30.1° ± 0.2° |
| 35 | 30.3° ± 0.2° |
| 36 | 30.8° ± 0.2° . |

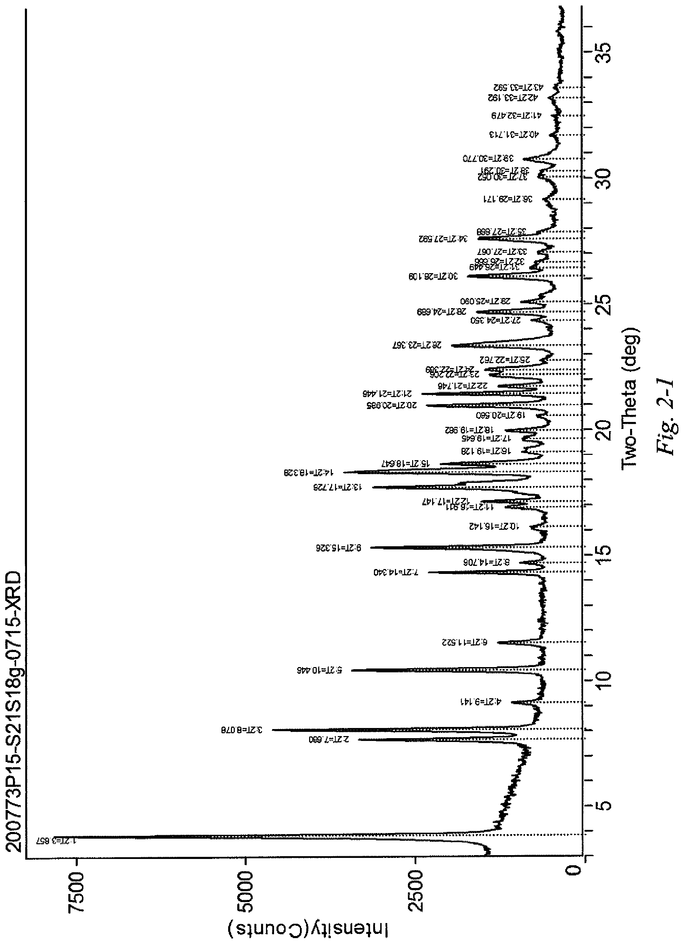

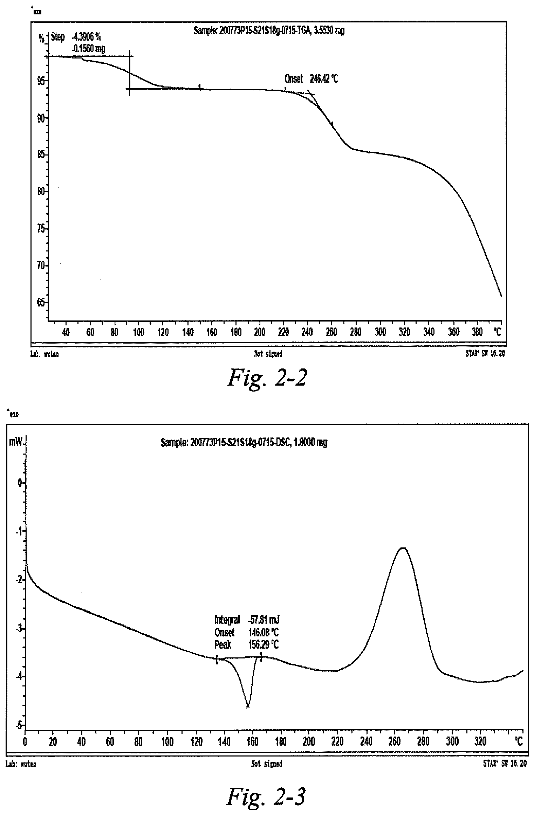

Furthermore, the crystal form II of the compound of formula (A) has an X-ray powder diffraction pattern substantially as shown in Figure 2-1.

Furthermore, the crystal form II of the compound of formula (A) is an ethyl ether solvate and has a decomposition temperature of 246°C ± 3°C.

Furthermore, the crystal form II of the compound of formula (A) has a desolvation peak between 130°C and 170°C, which is accompanied by melting.

Furthermore, the crystal form II of the compound of formula (A) has a TGA curve substantially as shown in Figure 2-2 and a DSC pattern substantially as shown in Figure 2-3.

The present application also provides a method for preparing the crystal form II of the compound of formula (A), which method comprises:
a) a magma crystallization process, which involves: at room temperature, adding a crude of the compound of formula (A) to a solvent to obtain a suspension, and stirring and separating the suspension to obtain the crystal form II; or
b) a diffusion crystallization process, which involves: taking and placing a crude of the compound of formula (A) in a centrifugal tube, and then placing same in a solvent atmosphere and leaving same to stand for a period of time to obtain the crystal form II.

The present application also provides a method for preparing the crystal form II of the compound of formula (A), which method comprises:
a) a magma crystallization process, which involves: at room temperature, adding a crude of the compound of formula (A) to a solvent to obtain a suspension, and stirring and separating the suspension to obtain the crystal form II; or
b) a diffusion crystallization process, which involves: taking and placing a crude of the compound of formula (A) in a centrifugal tube, and then placing same in a solvent atmosphere and leaving same to stand to obtain the crystal form II.

The present application also provides a method for preparing the crystal form II of the compound of formula (A), which method comprises:
a) at room temperature, mixing a crude of the compound of formula (A) with a solvent to obtain a suspension, and stirring and separating the suspension to obtain the crystal form II; or
b) placing a crude of the compound of formula (A) in a solvent atmosphere and leaving same to stand to obtain the crystal form II.

In some embodiments, according to the above-mentioned methods for preparing the crystal form II of the compound of formula (A), the solvent in a) is selected from a mixed solvent of an ester and ethyl ether, and preferably the ester is isopropyl acetate.

In some embodiments, according to the above-mentioned methods for preparing the crystal form II of the compound of formula (A), a volume ratio of isopropyl acetate to ethyl ether in a) is 1 : 2 - 1 : 4, preferably 1.5 : 5.

In some embodiments, according to the above-mentioned methods for preparing the crystal form II of the compound of formula (A), the solvent in b) is selected from ethyl ether.

The present application also provides a pharmaceutical composition, comprising a therapeutically effective amount of the crystal form I or crystal form II of the compound of formula (A) described above, and a pharmaceutically acceptable carrier and/or excipient.

Furthermore, the above-mentioned pharmaceutical composition also comprises one or more second therapeutic agents having an anti-influenza virus effect,
wherein preferably, the one or more second therapeutic agents having the anti-influenza virus effect are neuraminidase inhibitors or M2 ion channel blockers.

The present application further provides the use of the crystal form I or crystal form II of the compound of formula (A) or the composition described above in the preparation of a drug for preventing/treating influenza.

The present application further provides the crystal form I or crystal form II of the compound of formula (A) or the composition described above, for use in the treatment and/or prevention of influenza.

The present application further provides a method for treating and/or preventing influenza, which method comprises administering to a subject in need thereof a therapeutically effective amount of the crystal form I or crystal form II of the compound of formula (A) or the composition described above.

The present application further provides a composition for treating and/or preventing influenza, which composition comprises the crystal form I or crystal form II of the compound of formula (A) or the composition described above.

The crystal form I or crystal form II of the present application accounts for approximately 5 wt% to approximately 100 wt% of a bulk drug.

In some embodiments, the crystal form I or crystal form II of the present application accounts for approximately 10 wt% to approximately 100 wt% of the bulk drug.

In some embodiments, the crystal form I or crystal form II of the present application accounts for approximately 15 wt% to approximately 100 wt% of the bulk drug.

In some embodiments, the crystal form I or crystal form II of the present application accounts for approximately 20 wt% to approximately 100 wt% of the bulk drug.

In some embodiments, the crystal form I or crystal form II of the present application accounts for approximately 25 wt% to approximately 100 wt% of the bulk drug.

In some embodiments, the crystal form I or crystal form II of the present application accounts for approximately 30 wt% to approximately 100 wt% of the bulk drug.

In some embodiments, the crystal form I or crystal form II of the present application accounts for approximately 35 wt% to approximately 100 wt% of the bulk drug.

In some embodiments, the crystal form I or crystal form II of the present application accounts for approximately 40 wt% to approximately 100 wt% of the bulk drug.

In some embodiments, the crystal form I or crystal form II of the present application accounts for approximately 45 wt% to approximately 100 wt% of the bulk drug.

In some embodiments, the crystal form I or crystal form II of the present application accounts for approximately 50 wt% to approximately 100 wt% of the bulk drug.

In some embodiments, the crystal form I or crystal form II of the present application accounts for approximately 55 wt% to approximately 100 wt% of the bulk drug.

In some embodiments, the crystal form I or crystal form II of the present application accounts for approximately 60 wt% to approximately 100 wt% of the bulk drug.

In some embodiments, the crystal form I or crystal form II of the present application accounts for approximately 65 wt% to approximately 100 wt% of the bulk drug.

In some embodiments, the crystal form I or crystal form II of the present application accounts for approximately 70 wt% to approximately 100 wt% of the bulk drug.

In some embodiments, the crystal form I or crystal form II of the present application accounts for approximately 75 wt% to approximately 100 wt% of the bulk drug.

In some embodiments, the crystal form I or crystal form II of the present application accounts for approximately 80 wt% to approximately 100 wt% of the bulk drug.

In some embodiments, the crystal form I or crystal form II of the present application accounts for approximately 85 wt% to approximately 100 wt% of the bulk drug.

In some embodiments, the crystal form I or crystal form II of the present application accounts for approximately 90 wt% to approximately 100 wt% of the bulk drug.

In some embodiments, the crystal form I or crystal form II of the present application accounts for approximately 95 wt% to approximately 100 wt% of the bulk drug.

In some embodiments, the crystal form I or crystal form II of the present application accounts for approximately 98 wt% to approximately 100 wt% of the bulk drug.

In some embodiments, the crystal form I or crystal form II of the present application accounts for approximately 99 wt% to approximately 100 wt% of the bulk drug.

In some embodiments, the crystal form I or crystal form II of the present application substantially accounts for 100 wt% of the bulk drug, that is, the bulk drug is substantially a pure phase crystal.

It can be understood that, as is well known in the art of thermogravimetric analysis (TGA) and differential scanning calorimetry (DSC), melting peak heights of a TGA curve and a DSC curve depend on many factors related to sample preparation and geometric shapes of instruments, and peak positions are relatively insensitive to experiment details. Therefore, in some embodiments, the crystallized compounds of the present application have TGA and DSC patterns comprising characteristic peak positions, which have substantially the same properties as the TGA and DSC patterns provided in the drawings of the present application, with an error tolerance of measured values within ± 5°C, which is generally required to be within ± 3°C.

It can be understood that the numerical values described and claimed in the present application are approximate values. Changes in values may be attributed to device calibration, device errors, crystal purity, crystal size, sample size and other factors.

It can be understood that the crystal forms of the present application are not limited to the characteristic patterns such as XRD, DSC, TGA, DVS and sorption isotherm curve graphs, which are completely the same as those described in the drawings disclosed in the present application, and any crystal form having a characteristic pattern which is substantially or essentially the same as those described in the drawings falls within the scope of the present application.

The "therapeutically effective amount" means an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The "room temperature" refers to 10°C - 30°C, preferably with humidity > 30% RH.

As used in the present application, the expressions "substantially as shown in figure...", "substantially the same as" or "essentially the same as" for defining the figures have the same meaning, which are intended to mean that, in view of acceptable deviations in the art, a person skilled in the art would consider that the figures are the same as the reference figures. Such deviations may be caused by known factors in the art related to instruments, operating conditions, human factors, etc. For example, a person skilled in the art can appreciate that an endothermic start temperature and an endothermic peak temperature measured by differential scanning calorimetry (DSC) can vary significantly with experiments. In some embodiments, it is considered that two patterns are substantially the same when the change in the positions of characteristic peaks of the two patterns does not exceed ± 5%, ± 4%, ± 3%, ± 2% or ± 1%. For example, it would have readily occurred to a person skilled in the art to identify whether two X-ray diffraction patterns or two DSC patterns are substantially the same. In some embodiments, it is considered that X-ray diffraction patterns are substantially the same when the change in the 20 angle of characteristic peaks of the two X-ray diffraction patterns does not exceed ± 0.3°, ± 0.2° or ± 0.1°.

As used in the present application, the term "approximately" should be understood to be within a range of normal tolerance in the art, for example, "approximately" can be understood to be within ± 10%, ± 9%, ± 8%, ± 7%, ± 6%, ± 5%, ± 4%, ± 3%, ± 2%, ± 1%, ± 0.5%, ± 0.1%, ± 0.05% or ± 0.01% of the value. Unless otherwise obvious from the context, all numeric values provided by the present application are modified with the term "approximately".

According to the crystal form I or crystal form II of the compound of formula (A) of the present application, the specific dosage and use method for different patients depend on many factors, including the age, weight, gender, natural health status and nutritional status of a patient, the active intensity, taking time and metabolic rate of the compound, the severity of a disorder, and the subjective judgment of a diagnosing and treating physician. A dosage of 0.01-1000 mg/kg body weight/day is preferably used here.

The crystal forms disclosed in the present application have the following beneficial effects:
1. the crystal form I and crystal form II are easy to prepare, high in purity and bioavailability, and good in pressure stability, fluidity and solubility, which are especially suitable for the manufacture of various pharmaceutical dosage forms and industrial scale-up production;
2. compared with an amorphous form, the crystal form I especially also has the obvious advantages of good stability, high bioavailability and low hygroscopicity.

### Brief Description of the Drawings

Figure 1-1 shows an X-ray powder diffraction pattern of the crystal form I of the compound A, as determined by using Cu-Kα radiation;
Figure 1-2 shows a thermogravimetric analysis (TGA) curve of the crystal form I of the compound A;
Figure 1-3 shows a differential scanning calorimetry (DSC) curve of the crystal form I of the compound A;
Figure 1-4 shows a dynamic vapor sorption (DVS) curve of the crystal form I of the compound A;
Figure 1-5 shows a sorption isotherm curve of the crystal form I of the compound A;
Figure 1-6 shows a configuration diagram of the compound A based on single crystal X-ray analysis;
Figure 2-1 shows an X-ray powder diffraction pattern of the crystal form II of the compound A, as determined by using Cu-Kα radiation;
Figure 2-2 shows a thermogravimetric analysis (TGA) curve of the crystal form II of the compound A;
Figure 2-3 shows a differential scanning calorimetry (DSC) curve of the crystal form II of the compound A;

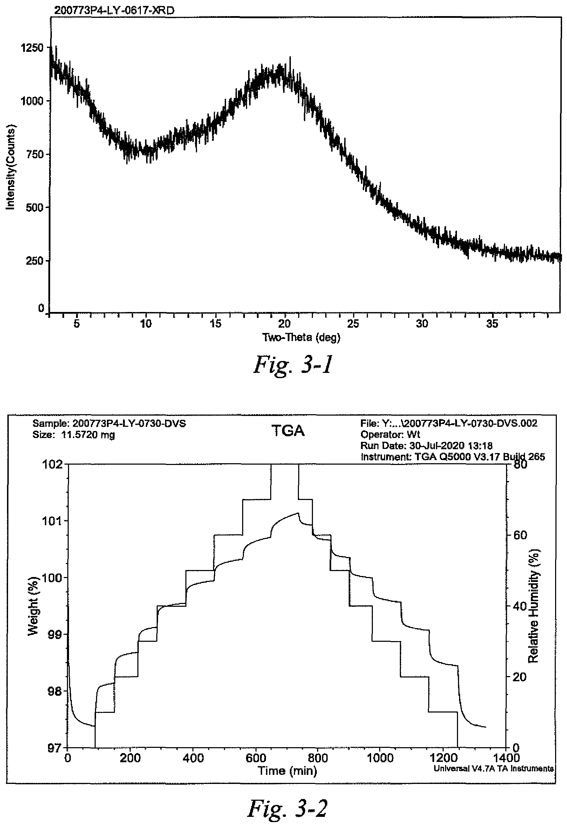

Figure 3-1 shows an X-ray powder diffraction pattern of the amorphous form of the compound A;
Figure 3-2 shows a dynamic vapor sorption (DVS) curve of the amorphous form of the compound A;

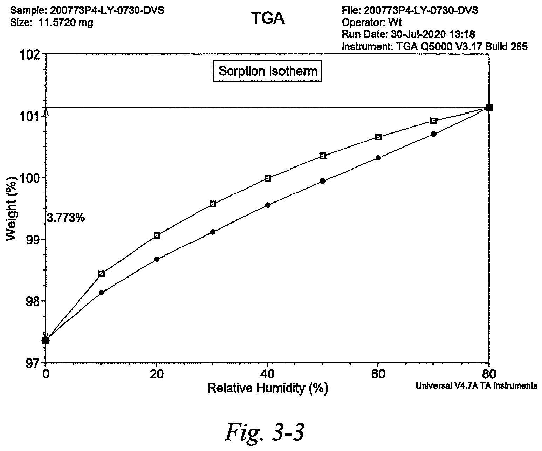

Figure 3-3 shows a sorption isotherm curve of the amorphous form of the compound A.

### Detailed Description of Embodiments

The content of the present application is described in detail with the following examples. If a specific condition is not indicated in the examples, a conventional condition is used in an experimental method. The listed examples are intended to better illustrate the content of the present application, but should not be construed as limiting the content of the present application. According to the above-mentioned content of the application, a person skilled in the art can make unsubstantial modifications and adjustments to the embodiments, which still fall within the scope of protection of the present application.

### Abbreviation definition

DDQ: 2,3-dichloro-5,6-dicyano-1,4-benzoquinone
EA: ethyl acetate
PE: petroleum ether
THF: tetrahydrofuran
DEAD: diethyl azodicarboxylate
DMF: N,N-dimethylformamide
HATU: 2-(7-azobenzotriazole)-N,N,N',N'-tetramethyluronium hexafluorophosphate
DIEA: N,N-diisopropylethylamine

### Detection method

The structure of the compound is determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvent for determination is deuterated dimethyl sulfoxide (DMSO-d6), deuterated chloroform (CDCl3) and deuterated methanol (CD3OD); and the internal standard is tetramethylsilane (TMS);
MS is determined with Agilent 6120B (ESI) and Agilent 6120B (APCI));
HPLC is determined with Agilent 1260DAD high pressure liquid chromatograph (Zorbax SB-C18 100 × 4.6 mm, 3.5 µM);
Yantai Huanghai HSGF254 or Qingdao GF254 silica gel plate is used as a thin layer chromatography silica plate, and the silica gel plate for the thin layer chromatography (TLC) is of the specification of 0.15 mm - 0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

| **X-ray powder diffractometer (XRPD) and hot-stage XRPD** | | | |
|---|---|---|---|
| Instrument | Model | Bruker D8 Advance Diffractometer | |
| | Number | LY-01-034 | |
| | Technical indicator | Kα radiation (40 kV, 40 mA) with a copper target wavelength of 1.54 Å, a 0-20 goniometer, nickel filtration, and a Lynxeye detector | |
| | Acquisition software | Diffrac Plus XRD Commander | |
| | Calibration material | Corundum (Al₂O₃) | |
| | Analysis software | MDI Jade | |
| Accessory | Nonreflective sample plate | Specification | 24.6 mm diameter × 1.0 mm thickness |
| | | Manufacturer | MTI corporation |
| | Variable-temperature hot stage | Manufacturer | Shanghai Weitu Instrument Technology Development Co., Ltd. |
| | | Material of sample plate | Copper plate |
| Parameter | Detection angle | 3° - 40° 20/3° - 30° 20 (hot-stage XRPD) | |
| | Step length | 0.02° 20 | |
| | Speed | 0.2s.step⁻¹ | |
| | Sample size to be detected | >2 mg | |
| | Note | Unless otherwise specified, samples are not subjected to grinding before detection | |
| | | | |

| **Differential scanning calorimeter (DSC)** | | | |
|---|---|---|---|
| Instrument | Model | DSC 3 | |
| | Number | LY-01-167 | |
| | Control software | STARe software | |
| | Analysis software | STARe software | |
| | Sample tray | Aluminium crucible (with a cover and with perforation) | |
| Parameter | Sample size to be detected | 0.5 mg - 5 mg | |
| | Protective gas | Nitrogen gas | |
| | Gas flow rate | 50 mL/min | |
| | Commonly used | Segment 1 Start temp 0.00°C End temp 350°C or 400.0°C | |
| | detection method | Heating rate 10.0 k/min | |
| | | | |

| **Thermal gravimetric analyser (TGA)** | | | |
|---|---|---|---|
| Instrument | Model | TGA/DSC 3+ | |
| | Number | LY-01-166 | |
| | Control software | STARe software | |
| | Analysis software | STARe software | |
| | Sample tray | 70 µL ceramic crucible | |
| Parameter | Sample size to be detected | 1 mg - 10 mg | |
| | Protective gas | Nitrogen gas | |
| | Gas flow rate | 50 mL/min | |
| | Commonly used detection method | Segment 1 (MaxRes) | |
| | | Start temp 25.0°C | |
| | | End temp 150.0°C | |
| | | Heating rate 10.0 k/min | |
| | | Segment 2 | |
| | | Start temp 150.0°C | |
| | | End temp 400.0°C | |
| | | Heating rate 10.0 k/min | |

### Example 1 Preparation of compound A

### Intermediate 1: methyl 4-(5-((tert-butoxycarbonyl)amino)benzo[d]oxazol-2-yl)picolinate (intermediate 1)

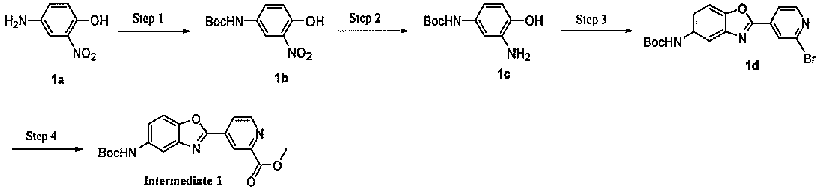

### Step 1: preparation of tert-butyl-(4-hydroxy-3-nitrophenyl)carbamate (1b)

Tetrahydrofuran (50 mL) and di-tert-butyl dicarbonate (10.6 g, 48.7 mmol) were successively added to known compound **1a** (5.0 g, 32.5 mmol). After the addition, the mixture was warmed to 70°C, reacted for 16 h and concentrated under reduced pressure to remove tetrahydrofuran. The resulting mixture was slurried with petroleum ether (100 mL) for 1 h and then filtered. The filter cake was collected and dried to obtain compound **1b** (6.1 g, 74%).

¹H NMR (400 MHz, CD₃OD) δ 8.25 (d, 1H), 7.56 (d, 1H), 7.06 (d, 1H) ,1.52 (s, 9H).

LC-MS (ESI): m/z =255.1[M+H]⁺.

### Step 2: preparation of tert-butyl-(3-amino-4-hydroxyphenyl)carbamate (1c)

At room temperature, compound **1b** (6.1 g, 24.0 mmol) was dissolved in anhydrous methanol (60 mL). Pd/C (2.1 g, with Pd content of 10% and water content of 50%) was added. Hydrogen was introduced. The mixture was warmed to 45°C and reacted for 5 h. After filtration, the filtrate was concentrated to obtain compound **1c** (4.3 g, 80%).

LC-MS (ESI): m/z =225.1[M+H]⁺.

### Step 3: preparation of tert-butyl (2-(2-bromopyridin-4-yl)-2,3-dihydrobenzo[d]oxazol-5-yl)carbamate (1d)

Compound **1c** (4.3 g, 19.2 mmol) was dissolved in methanol (50 mL). 2-bromopyridine-4-carboxaldehyde (3.6 g, 19.2 mmol) was added. The mixture was warmed to 70°C and stirred for 15 h. The reaction solution was cooled to room temperature and concentrated under reduced pressure to remove methanol. Then dichloromethane (200 mL) and DDQ (5.3 g, 23.0 mmol) were successively added to the residue. After the addition, the mixture was stirred for 2 h at room temperature, and a saturated aqueous sodium carbonate solution (100 mL) was added. The resulting solution was stirred for 10 min and filtered. The filtrate was extracted twice with dichloromethane (200 mL × 2). The combined organic phase was washed with water (100 mL), dried over anhydrous sodium sulphate and filtered. The filtrate was concentrated under reduced pressure, and then the residue was separated and purified by column chromatography (eluent: EA/PE = 10% - 50%) to obtain **1d** (4.1 g, 54%).

LC-MS (ESI): m/z =392.1[M+H]⁺.

### Step 4: preparation of methyl 4-(5-((tert-butoxycarbonyl)amino)benzo[d]oxazol-2-yl)picolinate (intermediate 1)

Methanol (25 mL), dichloromethane (25 mL), Pd(dppf)Cl₂ (804.0 mg, 1.1 mmol) and triethylamine (4.24 g, 42.0 mmol) were successively added to compound 1d (4.1 g, 10.5 mmol). Carbon monoxide was introduced; and then the reaction solution was warmed to 120°C and stirred for 14 h. The reaction solution was cooled to room temperature and then filtered. The filtrate was concentrated under reduced pressure, and then the residue was separated and purified by column chromatography (eluent: EA/PE = 10% - 50%) to obtain **intermediate** 1 (3.5 g, 90%).

¹H NMR (400 MHz, CDCl₃) & 8.95 (d, 1H), 8.89 (d, 1H), 8.26 (d, 1H), 7.86 (s, 1H), 7.54-7.47 (m, 2H) , 6.67 (s, 1H), 4.08 (s, 3H), 1.55 (s, 9H).

LC-MS (ESI): m/z = 370.1 [M+H]⁺.

### Intermediate 2: (R)-4-((5-methyl-2H-tetrazol-2-yl)(phenyl)methyl)piperidin-1-ium 2,2,2-trifluoroacetate (intermediate 2)

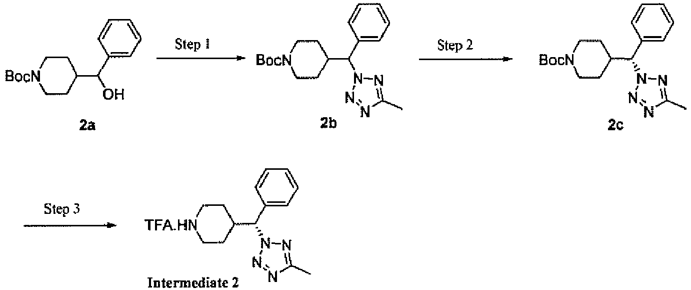

### Step 1: preparation of tert-butyl 4-((5-methyl-2H-tetrazol-2-yl)(phenyl)methyl)piperidine-1-carboxylate (2b)

At room temperature, **2a** (580 mg, 2.0 mmol), 5-methyltetrazole (185 mg, 2.2 mmol) and triphenylphosphine (787 mg, 3.0 mmol) were dissolved in anhydrous THF (20 mL). The mixture was cooled to 0°C under nitrogen protection, and then DEAD (520 mg, 3.0 mmol) was added dropwise. The mixture was allowed to naturally warm to room temperature, and reacted overnight. The reaction solution was concentrated under reduced pressure and subjected to column chromatography to obtain **2b** (440 mg, 61.0%).

LC-MS (ESI): m/z =358.3[M+H]⁺.

### Step 2: preparation of (R)-tert-butyl 4-((5-methyl-2H-tetrazol-2-yl)(phenyl)methyl)piperidine-1-carboxylate (2c)

Compound **2b** was resolved by chiral HPLC to obtain compound **2c** (tR = 1.78 min, 200 mg, 45.5%).

Resolution conditions were as follows:
instrument: MG II preparative SFC (SFC-1); column type: ChiralCel OJ, 250 × 30 mm I.D., 5 µm; mobile phase: A: CO₂, B: ethanol; gradient: B 15%; flow rate: 60 mL/min; back pressure: 100 bar; column temperature: 38°C; column length: 220 nm; time period: about 5 min; sample preparation: 0.44 g of compound **2b** was dissolved in a mixed solvent (4 mL) of dichloromethane and methanol; sample injection: 2 mL/injection.

### Step 3: preparation of (R)-4-((5-methyl-2H-tetrazol-2-yl)(phenyl)methyl)piperidin-1-ium 2,2,2-trifluoroacetate (intermediate 2)

At room temperature, **2c** (200 mg, 0.55 mmol) was dissolved in dichloromethane (10 mL), trifluoroacetic acid (2.5 mL) was added dropwise, and the mixture was stirred for another 2 h. The reaction solution was subjected to rotary evaporation to obtain a crude of **intermediate 2** (300 mg), which was directly used in the next reaction without purification.

LC-MS (ESI): m/z =258.2[M+H]⁺.

### Compound A: (1S,2S)-2-fluoro-N-(2-(2-(4-((R)-(5-methyl-2H-tetrazol-2-yl)(phenyl)methyl)piperidine-1-carbonyl)pyridin-4-yl)benzo[d]oxazol-5-yl)cyclopropane-1-carboxamide

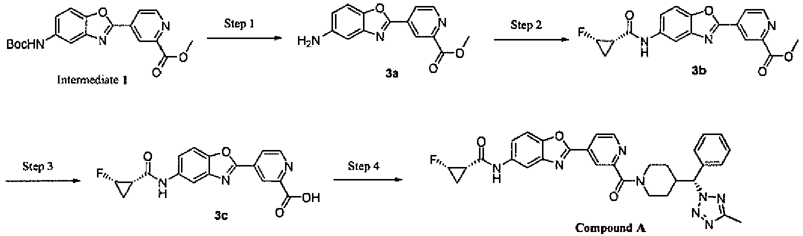

### Step 1: preparation of methyl-4-(5-aminobenzo[d]oxazol-2-yl)picolinate (3a)

**Intermediate 1** (600.0 mg, 1.62 mmol) was dissolved in dichloromethane (5 mL), and trifluoroacetic acid (2 mL) was added. After the addition, the mixture was stirred for 2 h at room temperature, adjusted to pH = 8-9 with a saturated aqueous sodium carbonate solution, and extracted twice with dichloromethane (50 mL × 2). The organic phases were combined, dried and filtered. The filtrate was concentrated to obtain compound **3a** (396.0 mg, 90%).

LC-MS (ESI): m/z =270.1[M+H]⁺.

### Step 2: preparation of methyl-4-(5-((1S,2S)-2-fluorocyclopropane-1-carboxamido)benzo[d]oxazol-2-yl)picolinate (3b)

DMF (50 mL), (1S,2S)-2-fluorocyclopropanecarboxylic acid (425 mg, 4.1 mmol), HATU (2.1 g, 5.58 mmol) and DIEA (1.44 g, 1 1 .16 mmol) were successively added to compound **3a** (1.0 g, 3.71 mmol), and the mixture was stirred at room temperature for 5 h. The reaction was quenched by adding water, extracted 3 times with ethyl acetate and washed twice with saturated brine. The organic phase was dried and concentrated. The residue was separated and purified by silica gel column chromatography (eluent: EAlPE = 1/2 (v/v)) to obtain **3b** (1.1 g, 83.4%).

LC-MS (ESI): m/z = 356.3 [M+H]⁺.

### Step 3: preparation of 4-(5-((1S,2S)-2-fluorocyclopropane-1-carboxamido)benzo[d]oxazol-2-yl)picolinic acid (3c)

At room temperature, compound **3b** (1 g, 3.1 mmol) was dissolved in methanol (15 mL), and lithium hydroxide (700 mg) was dissolved in 20 mL of pure water. An aqueous solution of lithium hydroxide was added to the reaction solution. The mixture was stirred at 40°C for 0.5 h and then adjusted to pH = 6-7 with 2N hydrochloric acid. A large amount of solid was precipitated out, filtered by suction and washed with water (10 mL × 3). The filter cake was dried at 50°C to obtain 3c (1.0 g, 94.6%).

LC-MS (ESI): m/z =342.1 [M+H]⁺.

### Step 3: preparation of (1S,2S)-2-fluoro-N-(2-(2-(4-((R)-(5-methyl-2H-tetrazol-2-yl)(phenyl)methyl)piperidine-1-carbonyl)pyridin-4-yl)benzo[d]oxazol-5-yl)cyclopropane-1-carboxamide (compound A)

At room temperature, **3c** (170 mg, 0.5 mmol) and DIPEA (130 mg, 1.0 mmol) were dissolved in DMF (5 mL), and then HATU (230 mg, 0.6 mmol) was added. The mixture was stirred for 3 min, and then intermediate **2** (300 mg, approximately 0.55 mmol) was added. The mixture was reacted for another 30 min at room temperature. 30 mL of water was added, and the reaction solution was extracted with ethyl acetate (30 mL × 3). The organic phases were combined, washed with saturated sodium chloride (30 mL × 1), dried over anhydrous sodium sulphate, and concentrated under reduced pressure, and then the residue was subjected to column chromatography (DCM : MeOH = 30 : 1 - 15 : 1 (v/v)) to obtain compound **A** (130 mg, 44.8%).

Compound 120 recited in PCT/CN 2020/110764 and the above prepared compound A are amorphous forms as characterized by XRPD, and the XRPD pattern is shown in Figure 3-1.

LC-MS (ESI): m/z = 581.3[M+H]⁺.

¹H NMR (400 MHz, CDCl₃) δ 8.74-8.71 (m, 1H), 8.31 (s, 1H), 8.12-8.10 (m, 1H), 8.01 (s, 1H), 7.90-7.87 (m, 1H), 7.56-7.51 (m, 4H), 7.41-7.31 (m, 3H), 5.55-5.52 (m, 1H), 4.93-4.75 (m, 2H), 3.91-3.88 (m, 1H), 3.20-3.11 (m, 1H), 2.91-2.80 (m, 2H), 2.56-2.50 (m, 3H), 1.94-1.84 (m, 2H), 1.61-1.23 (m, 5H).

### Experimental example

Experimental method: the cytopathic effect (CPE) method was used to test the antiviral activity of the compound A against influenza viruses, and the MDCK cytotoxicity was also determined. The compound was tested for 8 concentrations (double replicate wells). CCK-8 reagent was used to detect cell viability. MDCK cells were seeded at a certain density in a microplate and cultured overnight at 37°C and 5% CO₂. The compound and viruses were added the next day. Cell control (without compound treatment or virus infection) and virus infection control (cells infected with virus and without compound treatment) were provided. The final concentration of DMSO in the cell culture medium was 0.5%. The cells were cultured at 37°C and 5% CO₂ for 5 days until the rate at which a cytopathic effect appears in virus control wells reached 80%-95%. CCK-8 reagent was used to detect cell viability, and the raw data was used to calculate the antiviral activity of the compound. GraphPad Prism software was used to analyse dose response curves of the compound and calculate EC₅₀ values.

Anti-proliferative activity results of compound A against IFVA/Mal/302/54 and IFVA/Weiss/43 viruses

| Compound | EC₅₀ (IFVA/Mal/302/54) µM | EC₅₀ (IFVA/Weiss/43) µM |
|---|---|---|
| A | 0.014 | 0.003 |

Anti-proliferative activity results of compound A against IFV A/PR/8/34 virus

| Compound | EC₅₀ (IFV A/PR/8/34) µM |
|---|---|
| A | 0.012 |

Conclusion: the compound A exhibits a good anti-proliferative activity against IFVA/Mal/302/54, IFVA/Weiss/43 and IFV A/PR/8/34 viruses.

### Example 2 Preparation of crystal form I of compound A

Method I: 50 mg of a crude of the compound of formula (A) was added to a solvent to obtain a suspension. The suspension was stirred at the corresponding temperature for a period of time, centrifuged, and dried overnight in vacuum at room temperature to obtain a solid. The solid was characterized by XRPD. The obtained solid was the crystal form I (Form 1). Specific experimental conditions were as follows.

| **Number** | **Temperature (°C)** | **Solvent 1** | **Solvent 2** | **Solvent 1/Solvent 2 (mL)** | **Stirring time of magma (day)** | **Result analysis** |
|---|---|---|---|---|---|---|
| 1 | Room temperature | Isopropyl ether | - | 2.0 | 13 | Form 1 |
| 2 | 40 | Methyl tert-butyl ether | - | 2.0 | 13 | Form 1 |
| 3 | Room temperature | Methanol | Water | 0.210.2 | 13 | Form 1 |
| 4 | Room temperature | Ethanol | n-Heptane | 0.15/0.6 | 17 | Form 1 |
| 5 | 40 | Acetonitrile | Water | 0.5/0.5 | 17 | Form 1 |
| 6 | Room temperature | Toluene | n-Butyl acetate | 0.6/0.1 | 17 | Form 1 |
| 7 | Room temperature | Isopropyl acetate | Methyl tert-butyl ether | 0.2/0.6 | 6 | Form 1 |
| 8 | Room temperature | Toluene | Methyl tert-butyl ether | 0.2/0.6 | 6 | Form 1 |

Method II: 15 mg of a crude of the amorphous compound A was taken and placed in a centrifugal tube, and a corresponding solvent 1 was added and dissolved into a clear solution. Then the clear solution was placed in the solvent atmosphere of a solvent 2 and left to stand until a solid was precipitated out. The solid was centrifuged and dried overnight in vacuum at room temperature to obtain a dried solid. The dried solid was confirmed as the crystal form I (Form 1) by XRPD characterization. XRPD characterization was carried out. Experimental conditions were as follows.

| **Number** | **Mode** | **Solvent 1** | **Solvent 1 (mL)** | **Solvent 2** | **Result analysis** |
|---|---|---|---|---|---|
| 1 | Gas-liquid | n-Butyl acetate | 0.2 | Isopropyl ether | Form 1 |

After XRPD identification, the solids prepared by methods I and II are both the crystal form I. The XRPD peaks are listed in Table 1, and the XRPD pattern is shown in Figure 1-1. The TGA pattern is shown in Figure 1-2, indicating that the crystal form I has a slow loss of approximately 2.6% of weight before 150°C, is an anhydride (or a tunnel hydrate), and has a decomposition temperature of approximately 246°C. The DSC pattern is shown in Figure 1-3, with a melting point of approximately 216°C.

According to the preparation of a single crystal of the compound A,
approximately 10 mg of a Form 1 sample was taken and placed in a small glass bottle, 0.6 mL of methanol was added and dissolved into a clear solution, and then the clear solution was placed in an isopropyl ether atmosphere and diffused through small holes at room temperature to obtain a bulk crystal. A single crystal structure information table is shown in Table 2, a single crystal configuration is shown in Figure 1-6, and single crystal structure analysis data are shown in Table 3.

**Table 1 List of XRPD peaks of crystal form I**

| **2-Theta** | **D** | **Height** | **I%** | **Area** | **1%** |
|---|---|---|---|---|---|
| 4.077 | 21.6555 | 17983 | 100 | 139561 | 100 |
| 8.794 | 10.0476 | 153 | 0.9 | 974 | 0.7 |
| 10.064 | 8.7819 | 1445 | 8 | 10071 | 7.2 |
| 11.325 | 7.8065 | 255 | 1.4 | 2156 | 1.5 |
| 13.559 | 6.5251 | 92 | 0.5 | 372 | 0.3 |
| 14.248 | 6.211 | 348 | 1.9 | 3058 | 2.2 |
| 14.547 | 6.0842 | 274 | 1.5 | 4398 | 3.2 |
| 14.806 | 5.9783 | 925 | 5.1 | 10204 | 7.3 |
| 16.266 | 5.4449 | 370 | 2.1 | 3863 | 2.8 |
| 16.521 | 5.3612 | 209 | 1.2 | 1716 | 1.2 |
| 16.844 | 5.2593 | 204 | 1.1 | 1567 | 1.1 |
| 17.488 | 5.067 | 1668 | 9.3 | 12517 | 9 |
| 18.348 | 4.8312 | 338 | 1.9 | 5906 | 4.2 |
| 18.627 | 4.7596 | 356 | 2 | 5703 | 4.1 |
| 18,844 | 4.7053 | 104 | 0.6 | 740 | 0.5 |
| 19.509 | 4.5464 | 584 | 3.2 | 5746 | 4.1 |
| 20.367 | 4.3567 | 1961 | 10.9 | 19489 | 14 |
| 21.207 | 4.186 | 986 | 5.5 | 10807 | 7.7 |
| 21.892 | 4.0567 | 122 | 0.7 | 866 | 0.6 |
| 22.486 | 3.9508 | 178 | 1 | 2347 | 1.7 |
| 22.746 | 3.9062 | 563 | 3.1 | 7151 | 5.1 |
| 23.026 | 3.8594 | 195 | 1.1 | 1838 | 1.3 |
| 23.671 | 3.7556 | 1620 | 9 | 13766 | 9.9 |
| 24.011 | 3.7032 | 467 | 2.6 | 6312 | 4.5 |
| 24.41 | 3.6436 | 718 | 4 | 7596 | 5.4 |
| 25.27 | 3.5215 | 217 | 1.2 | 2414 | 1.7 |
| 25.551 | 3.4834 | 660 | 3.7 | 10084 | 7.2 |
| 25.833 | 3.446 | 206 | 1.1 | 3357 | 2.4 |
| 26.851 | 3.3176 | 536 | 3 | 5991 | 4.3 |
| 27.875 | 3.198 | 221 | 1.2 | 2267 | 1.6 |
| 28.415 | 3.1384 | 110 | 0.6 | 2474 | 1.8 |
| 28.692 | 3.1087 | 513 | 2.9 | 7252 | 5.2 |
| 29.888 | 2.987 | 238 | 1.3 | 2850 | 2 |
| 30.309 | 2.9465 | 101 | 0.6 | 1105 | 0.8 |

**Table 2 Single crystal structure information table**

| **Phase Data** | |
|---|---|
| Formula | C₃₁H₂₉FN₈O₃ |
| Formula Weight | 580.62 |
| Crystal system | Monoclinic |
| Space group | C2 |
| Cell parameters | a = 46.5183(13) Å b = 6.6246(3) A, c = 20.2880(5)Å |
| | α = γ = 90° β = 109.7650(10)° |
| Crystal axis ratios | a/b = 7.0221 b/c = 0.3265 c/a = 0.4361 |
| Z | 8 |
| Cell volume | 5883.7(3) Å³ |
| Theoretical density | 1.311 g/cm³ |
| Flack parameter | 0.05(16) |
| R₁ parameter | 0.1171 |
| WR₂ parameter | 0.2503 |
| GOOF = S parameter | 1.037 |
| R_{sigma} | 0.0666 |
| Rᵢₙₜ | 0.0875 |

**Table 3 Single crystal structure analysis data (Atomic Coordinates (× 104) and Equivalent Isotropic Displacement Parameters (Å2× 103) for Compound A. U(eq) is Defined as One Third of the Trace of the Orthogonalized Uij Tensor)**

| **Atom** | **x** | **y** | **z** | **U(eq)** |
|---|---|---|---|---|
| F(1) | 3930(2) | -846(11) | 8096(3) | 154(3) |
| F(2) | 6516(2) | 8380(20) | 7920(9) | 284(7) |
| N(1) | 4262(1) | 2145(10) | 6829(3) | 72(2) |
| N(2) | 4968(1) | 6034(10) | 5979(3) | 65(2) |
| N(3) | 5623(2) | 11537(11) | 5386(5) | 102(3) |
| N(4) | 5998(1) | 7317(12) | 5130(3) | 75(2) |
| N(5) | 6813(4) | 1920(30) | 5542(8) | 91(4) |
| N(6) | 6781(5) | 20(30) | 5345(10) | 115(5) |
| N(7) | 6927(5) | -300(40) | 4917(10) | 123(6) |
| N(8) | 6996(4) | 2890(30) | 5278(11) | 96(5) |
| C(23) | 6661(3) | 2780(30) | 5998(7) | 90(4) |
| C(24) | 6889(4) | 2970(30) | 6745(6) | 97(6) |
| C(25) | 6926(4) | 1230(20) | 7145(9) | 137(7) |
| C(26) | 7129(4) | 1200(30) | 7828(8) | 159(8) |
| C(27) | 7297(3) | 2920(30) | 8111(6) | 142(8) |
| C(28) | 7261(4) | 4670(30) | 7711(8) | 179(10) |
| C(29) | 7057(4) | 4690(20) | 7028(8) | 113(6) |
| C(30) | 7076(5) | 1450(40) | 4907(12) | 121(6) |
| C(31) | 7269(6) | 1770(50) | 4515(14) | 156(10) |
| C(23') | 6742(7) | 4250(50) | 5796(1 1) | 97(7) |
| C(24') | 6893(6) | 2790(40) | 5394(12) | 98(10) |
| C(25') | 6992(6) | 3590(30) | 4875(13) | 128(10) |
| C(26') | 7113(5) | 2330(40) | 4488(11) | 153(13) |
| C(27') | 7136(5) | 270(40) | 4620(12) | 123(8) |
| C(28') | 7037(6) | -540(30) | 5139(13) | 123(11) |
| C(29') | 6915(6) | 720(40) | 5526(11) | 109(9) |
| N(5') | 6915(7) | 4020(50) | 6501(11) | 132(9) |
| N(6') | 7057(10) | 5640(50) | 6825(16) | 202(13) |
| N(7') | 7185(9) | 5060(60) | 7456(17) | 196(13) |
| N(8') | 6905(10) | 2500(60) | 6915(17) | 137(9) |
| C(30') | 7070(7) | 3280(50) | 7555(11) | 141(8) |
| C(3 I') | 7236(11) | 3480(80) | 8348(13) | 240(20) |
| N(9) | 5733(1) | 7837(10) | 8179(3) | 63(2) |
| N(10) | 5025(1) | 4027(9) | 9029(3) | 60(2) |
| N(11) | 4401(2) | -1485(10) | 9698(4) | 78(2) |
| N(12) | 3983(2) | 2735(13) | 9836(3) | 86(2) |
| N(13) | 3133(2) | 7399(16) | 9453(4) | 100(2) |
| N(14) | 2962(2) | 6840(20) | 9820(6) | 133(4) |
| N(15) | 3185(3) | 9255(17) | 9462(7) | 156(5) |
| N(16) | 3040(4) | 10130(30) | 9861(11) | 188(8) |
| O(1) | 3981(2) | 3145(11) | 7488(4) | 124(3) |
| O(2) | 4975(1) | 8655(7) | 6708(2) | 64(1) |
| O(3) | 5718(1) | 9321(9) | 4252(2) | 80(2) |
| O(4) | 5887(2) | 7355(12) | 7248(3) | 110(2) |
| O(5) | 5018(1) | 1396(7) | 8328(2) | 66(1) |
| O(6) | 4244(1) | 769(10) | 10733(2) | 82(2) |
| C(1) | 3812(3) | -1374(17) | 7411(6) | 104(3) |
| C(2) | 3567(2) | -180(20) | 7001(6) | 117(4) |
| C(3) | 3876(2) | -23(15) | 6896(5) | 98(3) |
| C(4) | 4038(2) | 1934(14) | 7104(5) | 84(2) |
| C(5) | 4452(2) | 3862(12) | 6859(3) | 60(2) |
| C(6) | 4468(2) | 5478(12) | 7317(4) | 63(2) |
| C(7) | 4644(2) | 7165(13) | 7310(3) | 66(2) |
| C(8) | 4797(2) | 7147(11) | 6842(3) | 58(2) |
| C(9) | 4787(2) | 5559(11) | 6397(3) | 56(2) |
| C(10) | 4610(2) | 3870(12) | 6390(4) | 66(2) |
| C(11) | 5063(2) | 7825(12) | 6184(3) | 63(2) |
| C(12) | 5254(2) | 9122(12) | 5906(3) | 60(2) |
| C(13) | 5262(2) | 11176(13) | 6001(5) | 89(3) |
| C(14) | 5449(3) | 12285(16) | 5737(6) | 128(4) |
| C(15) | 5610(2) | 9558(11) | 5303(4) | 63(2) |
| C(16) | 5429(2) | 8286(11) | 5544(3) | 60(2) |
| C(17) | 5786(2) | 8710(12) | 4862(4) | 64(2) |
| C(18) | 6173(2) | 7263(16) | 5894(4) | 86(3) |
| C(19) | 6286(3) | 5330(20) | 6112(4) | 134(5) |
| C(20) | 6469(3) | 4440(20) | 5678(5) | 136(5) |
| C(21) | 6274(3) | 4478(19) | 4918(4) | 130(5) |
| C(22) | 6148(2) | 6425(17) | 4682(4) | 93(3) |
| C(32) | 6411(3) | 10120(30) | 8085(11) | 168(6) |
| C(33) | 6202(3) | 11210(20) | 7471(7) | 148(6) |
| C(34) | 6082(2) | 10200(15) | 7971(5) | 92(3) |
| C(35) | 5896(2) | 8343(14) | 7760(4) | 74(2) |
| C(36) | 5548(2) | 6139(11) | 8156(3) | 59(2) |
| C(37) | 5531(2) | 4489(13) | 7714(4) | 68(2) |
| C(38) | 5356(2) | 2810(13) | 7732(4) | 73(2) |
| C(39) | 5202(2) | 2869(11) | 8197(3) | 60(2) |
| C(40) | 5209(2) | 4497(11) | 8628(3) | 56(2) |
| C(41) | 5385(2) | 6159(11) | 8623(3) | 57(2) |
| C(42) | 4923(2) | 2225(10) | 8844(3) | 56(2) |
| C(43) | 4736(2) | 963(11) | 9110(3) | 56(2) |
| C(44) | 4755(2) | -1106(11) | 9084(4) | 66(2) |
| C(45) | 4584(2) | -2245(12) | 9379(5) | 86(2) |
| C(46) | 4546(2) | 1756(1 1) | 9443(4) | 64(2) |
| C(47) | 4378(2) | 533(11) | 9715(4) | 59(2) |
| C(48) | 4190(2) | 1311(12) | 10123(3) | 64(2) |
| C(49) | 3829(3) | 2958(19) | 9076(4) | 143(5) |
| C(50) | 3739(2) | 4896(17) | 8841(5) | 107(4) |
| C(51) | 3595(2) | 6219(18) | 9254(4) | 102(3) |
| C(52) | 3773(2) | 5908(17) | 10049(5) | 99(3) |
| C(53) | 3818(2) | 3784(15) | 10254(4) | 90(3) |
| C(54) | 3252(2) | 5864(16) | 9074(4) | 90(3) |
| C(55) | 3062(2) | 5947(19) | 8301(5) | 95(3) |
| C(56) | 2898(3) | 4320(30) | 7988(7) | 154(5) |
| C(57) | 2714(4) | 4320(40) | 7267(8) | 181(7) |
| C(58) | 2712(4) | 5990(40) | 6901(8) | 174(7) |
| C(59) | 2889(3) | 7750(40) | 7212(8) | 170(7) |
| C(60) | 3059(2) | 7630(20) | 7914(6) | 129(4) |
| C(61) | 2914(4) | 8650(40) | 10049(10) | 178(9) |
| C(62) | 2742(5) | 9040(50) | 10528(10) | 331(17) |

### Example 3 Preparation of crystal form II of compound A

Method I: 150 mg of an amorphous compound A was taken, and 1.5 mL of ethyl ether and 0.45 mL of isopropyl acetate were added. The magma was stirred at room temperature for 3 days, centrifuged, and dried overnight in vacuum at room temperature to obtain the crystal form II.

Method II: approximately 20 mg of the amorphous compound A was taken and placed in a centrifugal tube, and then placed in an ethyl ether atmosphere and left to stand to obtain the crystal form II.

The above crystal form was identified as a crystal form by XRPD and named as crystal form II (Form 2). The XRPD peaks of the crystal form II are listed in Table 4; the XRPD pattern is shown in Figure 2-1; the TGA pattern is shown in Figure 2-2, indicating that the crystal form II has a loss of approximately 4.4% of weight at a weight loss step before 150°C, approximately one third of an ethyl ether molecule (theoretically, one third of an ethyl ether molecule accounts for approximately 4.1%), and has a decomposition temperature of approximately 246°C; and the DSC pattern is shown in Figure 2-3, in which the crystal form II has a desolvation peak between 130°C and 170°C, accompanied by melting.

**Table 4 List of XRPD peaks of crystal form II**

| **2-Theta** | **D** | **Height** | **I%** | **Area** | **I%** |
|---|---|---|---|---|---|
| 3.857 | 22.8913 | 6539 | 100 | 53083 | 100 |
| 7.68 | 11.5022 | 2538 | 38.8 | 20394 | 38.4 |
| 8.078 | 10.9365 | 3867 | 59.1 | 26926 | 50.7 |
| 9.141 | 9.6662 | 431 | 6.6 | 3242 | 6.1 |
| 10.446 | 8.462 | 2835 | 43.4 | 19483 | 36.7 |
| 11.522 | 7.6739 | 658 | 10.1 | 5496 | 10.4 |
| 14.34 | 6.1716 | 1689 | 25.8 | 9430 | 17.8 |
| 14.706 | 6.0187 | 362 | 5.5 | 3149 | 5.9 |
| 15.326 | 5.7766 | 2552 | 39 | 19025 | 35.8 |
| 16.142 | 5.4862 | 205 | 3.1 | 1483 | 2.8 |
| 16.91 1 | 5.2387 | 491 | 7.5 | 3536 | 6.7 |
| 17.147 | 5.1668 | 793 | 12.1 | 5285 | 10 |
| 17.726 | 4.9996 | 2339 | 35.8 | 26058 | 49.1 |
| 18.328 | 4.8365 | 2897 | 44.3 | 35637 | 67.1 |
| 18.647 | 4.7545 | 1345 | 20.6 | 12325 | 23.2 |
| 19.128 | 4.6361 | 285 | 4.4 | 3309 | 6.2 |
| 19.645 | 4.5152 | 309 | 4.7 | 4601 | 8.7 |
| 19.982 | 4.4397 | 599 | 9.2 | 5412 | 10.2 |
| 20.56 | 4.3163 | 151 | 2.3 | 940 | 1.8 |
| 20.985 | 4.2298 | 1745 | 26.7 | 14428 | 27.2 |
| 21.446 | 4.14 | 1755 | 26.8 | 10731 | 20.2 |
| 21.746 | 4.0835 | 677 | 10.4 | 6359 | 12 |
| 22.206 | 3.9999 | 855 | 13.1 | 27079 | 51 |
| 22.389 | 3.9676 | 863 | 13.2 | 16948 | 31.9 |
| 22.762 | 3.9034 | 80 | 1.2 | 221 | 0.4 |
| 23.367 | 3.8037 | 1467 | 22.4 | 19225 | 36.2 |
| 24.35 | 3.6524 | 237 | 3.6 | 1726 | 3.3 |
| 24.689 | 3.603 | 1110 | 17 | 12848 | 24.2 |
| 25.09 | 3.5464 | 471 | 7.2 | 8005 | 15.1 |
| 26.109 | 3.4101 | 1206 | 18.4 | 11975 | 22.6 |
| 26.449 | 3.3672 | 315 | 4.8 | 9110 | 17.2 |
| 26.666 | 3.3402 | 236 | 3.6 | 9331 | 17.6 |
| 27.067 | 3.2916 | 137 | 2.1 | 687 | 1.3 |
| 27.592 | 3.2301 | 1063 | 16.3 | 12628 | 23.8 |
| 27.868 | 3.1988 | 223 | 3.4 | 2635 | 5 |
| 29.171 | 3.0588 | 183 | 2.8 | 2817 | 5.3 |
| 30.052 | 2.9711 | 232 | 3.5 | 4245 | 8 |
| 30.291 | 2.9482 | 233 | 3.6 | 7141 | 13.5 |
| 30.77 | 2.9034 | 450 | 6.9 | 5469 | 10.3 |
| 31.713 | 2.8191 | 126 | 1.9 | 1586 | 3 |
| 32.479 | 2.7544 | 94 | 1.4 | 353 | 0.7 |
| 33.192 | 2.6969 | 162 | 2.5 | 3180 | 6 |
| 33.592 | 2.6657 | 90 | 1.4 | 1320 | 2.5 |

### Example 4 Experiment of crystal form transformation of crystal form I of compound A

According to the experiment of crystal form transformation in water, the experimental scheme and results are shown in Table 5.

**Table 5 Experimental information table of crystal form transformation in water**

| **Item** | **Content** | |
|---|---|---|
| Crystal form | Form 1 | |
| Experimental condition | Temperature | Room temperature |
| | Solvent | Water |
| Stirring of magma at room temperature for 7 days | Form 1 | |
| Experiment result | The crystal form of Form 1 remained unchanged after the magma was stirred in water for 7 days | |

### Example 5 Stability experiment of crystal form I of compound A

The stability experiment scheme and results are shown in Table 6.

**Table 6 Stability experiment information table of crystal form**

| **Item** | **Content** | | | |
|---|---|---|---|---|
| Crystal form | Form 1 | | | |
| Sample amount | 20 mg | | | |
| Sample batch | 200773P21-S20h | | | |
| Experimental operation | Spreading in a sample bottle | | | |
| Detection time | Day 0, day 10 | | | |
| Detection item | Crystal form (XRPD detection) and melting point (DSC detection) | | | |
| Experimental condition | Long time (25°C ± 2°C, 65% RH ± 10% RH, being open and in a dark place) | Acceleration (40°C ± 2°C, 75% RH ± 10% RH, being open and in a dark place) | High humidity (25°C ± 2°C, 85% RH ± 10% RH, being open and in a dark place) | High temperature (40°C ± 2°C, being sealed and in a dark place) |
| Experiment result | Neither the crystal form nor the melting point changes significantly after Form 1 was stored for 10 days under the condition of long time, acceleration, high temperature or high humidity | | | |

Experimental conclusion: the crystal form I of the compound A has a good stability.

### Example 6 Hygroscopicity experiment of crystal form I of compound A

Experimental condition:

| **Dynamic vapor sorption (DVS)** | | |
|---|---|---|
| Instrument | Model | TA Instruments Q5000TGA |
| | Number | LY-01-004 |
| | Control software | Thermal Advantage |

| | | |
|---|---|---|
| | Analysis software | Universal Analysis |
| | Sample tray | Platinum crucible |
| Parameter | Sample size to be detected | 1 mg - 10 mg |
| | Protective gas | Nitrogen gas |
| | Gas flow rate | 10 mL/min |
| | Commonly used detection method | Equilibrate at 25°C; Humidity 0%; Isothermal for 90 min; Abort next iso if weight (%) < 0.0100 for 15.00 min; step humidity 10% every 90 min to 80.00%; Abort next iso if weight (%) < 0.0100 for 15.00 min; step humidity 10% every 90 min to 0.00% |
| Determination criteria | Not or almost not hygroscopic | Weight gain of less than 0.2% |
| | Slightly hygroscopic | Weight gain of less than 2% but no less than 0.2% |
| | Hygroscopic | Weight gain of less than 15% but no less than 2% |
| | Extremely hygroscopic | Weight gain of no less than 15% |
| | Deliquescence | Absorbing enough moisture to form a liquid |

The DVS pattern is shown in Figure 1-4; and the sorption isotherm curve is shown in Figure 1-5. The results show that a weight change of the crystal form I in a range of 0% RH to 80% RH is approximately 0.3%; a weight change of the amorphous form of the compound A in a range of 0% RH to 80% RH is approximately 3.8% (Figure 3-2 and Figure 3-3); and compared with the amorphous form, the crystal form I has a significantly lower hygroscopicity.

## Claims

1. A crystal form I of a compound of formula (A), **characterized in that** the crystal form I has an X-ray powder diffraction pattern comprising characteristic diffraction peaks at 4.1° ± 0.2°, 10.1° ± 0.2°, 14.8° ± 0.2°, 17.5° ± 0.2°, 20.4° ± 0.2°, 21.2° ± 0.2°, and 23.7° ± 0.2° 2θ, as determined by using Cu-Kα radiation,

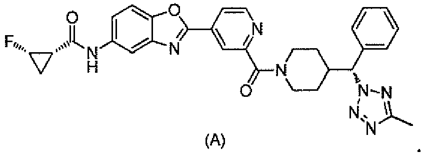

2. The crystal form I of claim 1, **characterized in that** the crystal form I has an X-ray powder diffraction pattern further comprising characteristic diffraction peaks at 19.5° ± 0.2°, 22.7° ± 0.2°, 24.4° ± 0.2°, 25.6° ± 0.2°, 26.9° ± 0.2°, and 28.7° ± 0.2° 2θ.

3. The crystal form I of claim 2, **characterized in that** the crystal form I has an X-ray powder diffraction pattern further comprising characteristic diffraction peaks at 14.2° ± 0.2°, 16.3° ± 0.2°, 18.3° ± 0.2°, 18.6° ± 0.2°, and 24.0° ± 0.2° 2Θ.

4. The crystal form I of claim 1, **characterized in that** the crystal form I has an X-ray powder diffraction pattern comprising characteristic diffraction peaks at 20 in the table below:
| Number | 20 |
|---|---|
| 1 | 4.1° ± 0.2° |
| 2 | 10.1° ± 0.2° |
| 3 | 11.3° ± 0.2° |
| 4 | 14.2° ± 0.2° |
| 5 | 14.8° ± 0.2° |
| 6 | 16.3° ± 0.2° |
| 7 | 16.5° ± 0.2° |
| 8 | 16.8° ± 0.2° |
| 9 | 17.5° ± 0.2° |
| 10 | 18.3° ± 0.2° |
| 11 | 18.6° ± 0.2° |
| 12 | 19.5° ±0.2° |
| 13 | 20.4° ± 0.2° |
| 14 | 21.2° ± 0.2° |
| 15 | 22.7° ± 0.2° |
| 16 | 23.7° ± 0.2° |
| 17 | 24.0° ± 0.2° |
| 18 | 24.4° ± 0.2° |
| 19 | 25.6° ± 0.2° |
| 20 | 26.9° ±0.2° |
| 21 | 27.9° ± 0.2° |
| 22 | 28.7° ±0.2° |
| 23 | 29.9° ± 0.2° |

5. The crystal form I of claim 1, **characterized in that** the crystal form I has an X-ray powder diffraction pattern substantially as shown in Figure 1-1.

6. A method for preparing the crystal form I of any one of claims 1-5, comprising
1) at 4°C - 40°C, mixing a crude of the compound of formula (A) with a solvent to obtain a suspension, and stirring and separating the suspension to obtain the crystal form I; or
2) mixing a crude of the compound of formula (A) with a solvent 1 and dissolving into a clear solution, placing the clear solution in the solvent atmosphere of a solvent 2 and leaving same to stand, precipitating out a solid, centrifuging and drying to obtain the crystal form I.

7. The preparation method of claim 6, **characterized in that** the solvent in 1) is a single-solvent system or a double-solvent mixed system, the single-solvent system is selected from one of isopropyl ether and methyl tert-butyl ether, the double-solvent mixed system comprises a first solvent and a second solvent, the first solvent is selected from one of methanol, ethanol, acetonitrile, toluene and isopropyl acetate, the second solvent is selected from one of water, n-heptane, n-butyl acetate and methyl tert-butyl ether, and preferably, the double-solvent mixed system is a methanol-water mixed solution, an ethanol-n-heptane mixed solution, an acetonitrile-water mixed solution, a toluene-n-butyl acetate mixed solution, an isopropyl acetate-methyl tert-butyl ether mixed solution or a toluene-methyl tert-butyl ether mixed solution.

8. The preparation method of claim 6, **characterized in that** the solvent 1 in 2) is an ester solvent, preferably n-butyl acetate, and the solvent 2 is an ether solvent, preferably isopropyl ether.

9. A crystal form II of the compound of formula (A), **characterized in that** the crystal form II has an X-ray powder diffraction pattern comprising characteristic diffraction peaks at 3.9° ± 0.2°, 7.7° ± 0.2°, 8.1° ± 0.2°, 10.4° ± 0.2°, 14.3° ± 0.2°, 15.3° ± 0.2°, 17.7°± 0.2°, 18.3° ± 0.2°, 18.6° ± 0.2°, 21.0° ± 0.2°, 21.4° ± 0.2°, 23.4° ± 0.2°, 24.7° ± 0.2°, 26.1° ± 0.2°, and 27.6° ± 0.2° 20, as determined by using Cu-Kα radiation.

10. The crystal form II of claim 9, **characterized in that** the crystal form II has an X-ray powder diffraction pattern further comprising characteristic diffraction peaks at 11.5° ± 0.2°, 16.9° ± 0.2°, 17.1° ± 0.2°, 20.0° ± 0.2°, 21.7° ± 0.2°, 22.2° ± 0.2°, 22.4° ± 0.2°, 25.1° ± 0.2°, and 30.8° ± 0.2° 2θ.

11. The crystal form II of claim 10, **characterized in that** the crystal form II has an X-ray powder diffraction pattern further comprising characteristic diffraction peaks at 9.1° ± 0.2°, 14.7° ± 0.2°, 19.1° ± 0.2°, 19.6° ± 0.2°, 24.4° ± 0.2°, 26.4° ± 0.2°, 26.7° ± 0.2°, 27.1° ± 0.2°, 30.1° ± 0.2°, and 30.3° ± 0.2° 2Θ.

12. The crystal form II of claim 9, **characterized in that** the crystal form II has an X-ray powder diffraction pattern comprising characteristic diffraction peaks at 2θ in the table below:
| Number | 2θ |
|---|---|
| 1 | 3.9° ± 0.2° |
| 2 | 7.7° ± 0.2° |
| 3 | 8.1° ± 0.2° |
| 4 | 9.1° ± 0.2° |
| 5 | 10.4° ± 0.2° |
| 6 | 11.5° ± 0.2° |
| 7 | 14.3° ±0.2° |
| 8 | 14.7° ± 0.2° |
| 9 | 15.3° ± 0.2° |
| 10 | 16.1° ± 0.2° |
| 11 | 16.9° ± 0.2° |
| 12 | 17.1° ± 0.2° |
| 13 | 17.7° ± 0.2° |
| 14 | 18.3° ± 0.2° |
| 15 | 18.6° ± 0.2° |
| 16 | 19.1° ± 0.2° |
| 17 | 19.6° ± 0.2° |
| 18 | 20.0° ± 0.2° |
| 19 | 20.6° ± 0.2° |
| 20 | 21.0° ± 0.2° |
| 21 | 21.4° ± 0.2° |
| 22 | 21.7° ± 0.2° |
| 23 | 22.2° ± 0.2° |
| 24 | 22.4° ± 0.2° |
| 25 | 23.4° ± 0.2° |
| 26 | 24.4° ± 0.2° |
| 27 | 24.7° ± 0.2° |
| 28 | 25.1° ± 0.2° |
| 29 | 26 1° ± 0.2° |
| 30 | 26.4° ± 0.2° |
| 31 | 26.7° ± 0.2° |
| 32 | 27.1° ± 0.2° |
| 33 | 27.6° ± 0.2° |
| 34 | 30.1° ± 0.2° |
| 35 | 30.3° ± 0.2° |
| 36 | 30.8° ± 0.2° |

13. The crystal form II of claim 9, **characterized in that** the crystal form II has an X-ray powder diffraction pattern substantially as shown in Figure 2-1.

14. A method for preparing the crystal form II of any one of claims 9-13, comprising:
a) at room temperature, mixing a crude of the compound of formula (A) with a solvent to obtain a suspension, and stirring and separating the suspension to obtain the crystal form II; or
b) placing a crude of the compound of formula (A) in a solvent atmosphere and leaving same to stand to obtain the crystal form II.

15. The preparation method of claim 14, **characterized in that** the solvent in a) is a mixed solvent of an ester solvent and ethyl ether, and preferably, the ester solvent is isopropyl acetate; and the solvent in b) is ethyl ether.

16. A pharmaceutical composition, comprising a therapeutically effective amount of the crystal form I of any one of claims 1-5 or the crystal form II of any one of claims 9-13, and a pharmaceutically acceptable carrier and/or excipient.

17. The pharmaceutical composition of claim 16, **characterized in that** the pharmaceutical composition further comprises one or more second therapeutic agents having an anti-influenza virus effect, and preferably, the second therapeutic agent is a neuraminidase inhibitor or an M2 ion channel blocker.

18. Use of the crystal form I of any one of claims 1-5, or the crystal form II of any one of claims 9-13, or the composition of claim 16 or 17 in the preparation of a drug for treating and/or preventing influenza.

19. The crystal form I of any one of claims 1-5, or the crystal form II of any one of claims 9-13, or the composition of claim 16 or 17 for use in the treatment and/or prevention of influenza.

20. A method for treating and/or preventing influenza, comprising administering to a subject in need thereof a therapeutically effective amount of the crystal form I of any one of claims 1-5, or the crystal form II of any one of claims 9-13, or the composition of claim 16 or 17.

21. A composition for treating and/or preventing influenza, comprising the crystal form I of any one of claims 1-5 or the crystal form II of any one of claims 9-13.
